# EUROPEAN PATENT APPLICATION

(11) **EP 4 772 136 A1**
(43) Date of publication of application: **08.07.2026**
(21) Application number: 24860104.9
(22) Date of filing: 21.06.2024
(51) Int. Cl.: A61C 9/00, A61B 5/00, G06T 1/00, G06T 19/20, G06F 3/14, G06F 17/00

(54) **METHOD AND SYSTEM FOR PROVIDING VISUAL GUIDE FOR ORAL SCANNER**

(30) Priority: 31.08.2023 KR 20230115844
(71) Applicant: Arcreal Inc., Seoul 06180 (KR)
(72) Inventor: JEON, Seung Hyun, Seoul 06180 (KR); KIM, Kyong Kook, Seoul 06180 (KR); CHOI, Yoon Soo, Seoul 06180 (KR)
(74) Representative: Studio Torta S.p.A.
(86) International application number: PCT/KR2024/008647
(87) International publication number: WO 2025/048180

(57) **Abstract**

The present disclosure provides a method of providing a visual guide for an oral scanner that is performed by at least one processor. The method includes obtaining first oral scan data from an oral scanner, calculating scan completeness of a plurality of regions of the first oral scan data, determining a supplementation-required region among the plurality of regions based on the calculated scan completeness, calculating a position and an orientation of the oral scanner relative to the supplementation-required region, and outputting, on a display, a visual guide generated based on first scan completeness of the supplementation-required region, the calculated position of the oral scanner, and the calculated orientation of the oral scanner.

## Description

### [Technical Field]

The present disclosure relates to a method and system for providing a visual guide for an oral scanner, and more particularly, to a method and system for calculating scan completeness of a plurality of regions of oral scan data obtained from an oral scanner and outputting, on a display, a visual guide indicating a position and an orientation of the oral scanner for a supplementation-required region determined based on the calculated scan completeness.

### [Background Art]

In dental clinics or the like, a dental gypsum model of a patient is prepared to perform treatment of a damaged tooth of the patient. However, in a process of preparing such a gypsum model, problems occur due to material consumption, cross-infection, damage to the prepared model, etc. To address this, a three-dimensional oral scanner that can obtain three-dimensional oral data has been developed in recent years.

Meanwhile, the three-dimensional oral scanner does not always guarantee sound three-dimensional oral scan data. Accordingly, there is a need to perform an oral scan repeatedly. However, for a user performing an oral scan, it is difficult to determine which site needs an oral scan for which reason. Accordingly, there is a problem that it is difficult to improve precision of oral data even when an oral scan is performed repeatedly.

### [Disclosure]

### [Technical Problem]

To address the above-described problems the present disclosure provides a method and device (system) for providing a visual guide for an oral scanner.

### [Technical Solution]

The present disclosure can be implemented in various ways, including a method, a device (system), or a computer program stored in a readable storage medium.

According to one embodiment of the present disclosure, a method of providing a visual guide may include obtaining first oral scan data from an oral scanner, calculating scan completeness of a plurality of regions of the first oral scan data, determining a supplementation-required region among the plurality of regions based on the calculated scan completeness, calculating a position and an orientation of the oral scanner relative to the supplementation-required region, and outputting, on a display, a visual guide generated based on first scan completeness of the supplementation-required region, the calculated position of the oral scanner, and the calculated orientation of the oral scanner.

A computer program stored in a computer-readable recording medium may be provided to execute the method of providing a visual guide according to one embodiment of the present disclosure in a computer.

According to one embodiment of the present disclosure, there is provided a device. The device may include a communication module, a memory, a display, and at least one processor connected to the memory and configured to execute at least one program that is included in the memory and computer-readable, wherein the at least one program may include instructions for obtaining first oral scan data from an oral scanner, calculating scan completeness of a plurality of regions of the first oral scan data, determining a supplementation-required region among the plurality of regions based on the calculated scan completeness, calculating a position and an orientation of the oral scanner relative to the supplementation-required region, and outputting, on the display, a visual guide generated based on first scan completeness of the supplementation-required region, the calculated position of the oral scanner, and the calculated orientation of the oral scanner.

### [Advantageous Effects]

According to some embodiments of the present disclosure, a user can conveniently check an intraoral region that requires an additional scan due to low scan completeness. Also, the user can easily check the type of supplementation-required region through a visual guide. Accordingly, the user can obtain more precise oral scan data by performing an additional scan.

According to some embodiments of the present disclosure, just by looking at a geometrical shape, a user can easily recognize for which reason a corresponding region requires supplementation. Also, from a surface size of the geometrical shape, the user can predict until when additional scanning should be performed. Accordingly, the user can easily perform an additional oral scan for a patient and can obtain high-quality oral scan data.

According to some embodiments of the present disclosure, a user can easily identify a tooth that requires an additional scan. Also, from a color or the like of an outline, which type of supplementation-required region is present on a corresponding tooth can be easily checked.

According to some embodiments of the present disclosure, by processing teeth that are not subject to scanning to be transparent, a user can focus more on a tooth that requires an additional scan. Also, by setting transparency for each type of supplementation-required region, the user can perform an additional scan while unnecessary supplementation-required regions are filtered out.

According to some embodiments of the present disclosure, from a color of an outline of a visual guide, a user can easily determine whether the current position and orientation of an oral scanner are appropriate. Accordingly, an additional oral scan can be conveniently performed.

Advantageous effects of the present disclosure are not limited to those mentioned above, and other unmentioned advantageous effects should be clearly understood by those of ordinary skill in the art to which the present disclosure pertains (hereinafter referred to as "person of ordinary skill") from the claims.

### [Description of Drawings]

Embodiments of the present disclosure will be described with reference to the accompanying drawings described below, in which like reference numerals indicate like elements but without being limited thereto.
FIG. 1 is a view showing a configuration in which an oral scanner is connected to a computing device according to one embodiment of the present disclosure.
FIG. 2 is a block diagram showing an internal configuration of the computing device connected to the oral scanner according to one embodiment of the present disclosure.
FIG. 3 is a flowchart showing an example of a method of providing a visual guide according to one embodiment of the present disclosure.
FIG. 4 is a view showing an example of outputting a visual guide according to one embodiment of the present disclosure.
FIG. 5 is a view showing examples of visual guides according to one embodiment of the present disclosure.
FIG. 6 is a view showing an example of outputting outlines of teeth according to one embodiment of the present disclosure.
FIG. 7 is a view showing an example of adjusting transparency of some teeth according to one embodiment of the present disclosure.
FIG. 8 is a view showing an example of changing the size of a geometrical shape according to one embodiment of the present disclosure.
FIG. 9 is a view showing an example of outputting together a visual guide indicating the current position and orientation of the oral scanner and a visual guide indicating a target position and orientation of the oral scanner on a display according to one embodiment of the present disclosure.
FIG. 10 is a view showing an example of a user interface screen on which a visual guide is output according to one embodiment of the present disclosure.
FIG. 11 is a view showing an example of outputting a visual guide according to one embodiment of the present disclosure.
FIG. 12 is a flowchart showing an example of a method according to one embodiment of the present disclosure.

### [Modes of the Invention]

Hereinafter, specific details for carrying out the present disclosure will be described in detail with reference to the accompanying drawings. However, in the following description, detailed description of a widely known function or configuration will be omitted if there is concern that the detailed description may unnecessarily obscure the gist of the present disclosure.

**In** the accompanying drawings, the same or corresponding components are denoted by the same reference numerals. Also, in the following description of embodiments, repeated description of the same or corresponding components may be omitted. However, even when description of a certain component is omitted, it is not intended to mean that the component is not included in a certain embodiment.

The advantages and features of disclosed embodiments and a method of achieving them will become apparent by referring to the embodiments described below with reference to the accompanying drawings. However, the present disclosure is not limited to the embodiments disclosed below and can be implemented in various different forms.

**In** the present specification, a singular expression includes a plural expression unless the context clearly indicates singularity. Also, a plural expression includes a singular expression unless the context clearly indicates plurality. Throughout the specification, when a certain part is described as including a certain component, this indicates that the certain part may further include other components instead of excluding other components unless particularly stated otherwise.

In the present disclosure, "display" may refer to any display device associated with a computing device, and may refer to, for example, any display device controlled by the computing device or capable of displaying any information/data provided from the computing device.

In the present disclosure, "each of a plurality of A's" or "a plurality of A's each" may refer to each of all components included in the plurality of A's, or may refer to each of some components included in the plurality of A's.

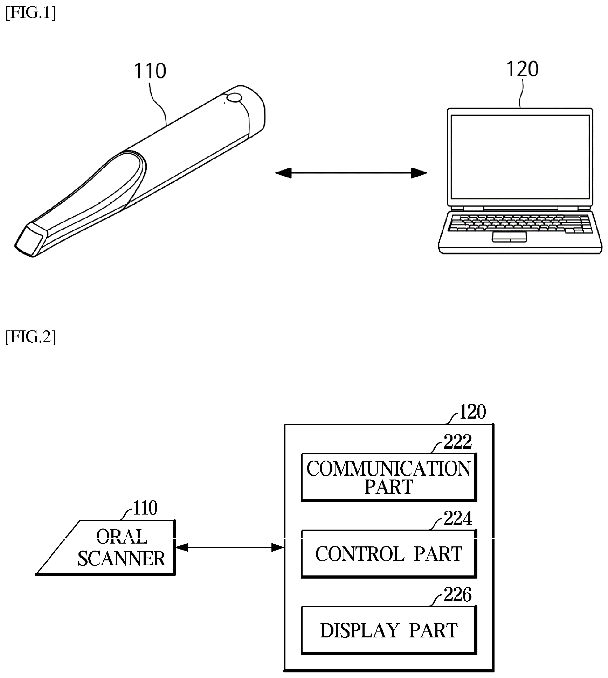

FIG. 1 is a view showing a configuration in which an oral scanner 110 is connected to a computing device 120 according to one embodiment of the present disclosure. The oral scanner 110 may, for example, be inserted into an oral cavity of a dental patient by a dentist and scan teeth in a non-contact manner to capture a plurality of pieces of two-dimensional image data. Also, the oral scanner 110 may send the plurality of captured pieces of two-dimensional image data to the computing device 120 or may execute three-dimensional oral structure modeling based on the pieces of two-dimensional image data by itself.

In one embodiment, the oral scanner 110 may be connected to the computing device 120 through a network configured to be capable of wired or wireless communication. Here, depending on an installation environment, the network may be constituted by a wired network, such as an electrical connection line including a copper cable, Ethernet, a wired home network (power line communication), telephone-line communication devices, or RS-serial communication; a wireless network, such as a mobile communication network, a wireless local area network (WLAN), Wi-Fi, Bluetooth, or ZigBee; or a combination thereof.

In one embodiment, the oral scanner 110 may exchange information and/or data such as two-dimensional image data and three-dimensional oral structure model data with the computing device 120. The oral scanner 110 and the computing device 120 may be configured to be physically separated as illustrated, but the present disclosure is not limited thereto. For example, the oral scanner 110 and the computing device 120 may be configured to be integrated in a single computing device.

In one embodiment, the computing device 120 may perform three-dimensional oral structure modeling by analyzing at least two pieces of two-dimensional image data or at least two stereo images obtained from the oral scanner 110. In order to perform such a function, the computing device 120 may include a processor (e.g., a central processing unit (CPU), a graphical processing unit (GPU), etc.) that can perform image processing and three-dimensional modeling and a memory that can store two-dimensional image data or three-dimensional oral structure model data.

FIG. 2 is a block diagram showing an internal configuration of the computing device 120 connected to the oral scanner 110 according to one embodiment of the present disclosure. In one embodiment, as illustrated, the computing device 120 may include a communication part 222, a control part 224, and a display part 226. The communication part 222 may be configured to transmit/receive information and/or data to/from the oral scanner 110. Specifically, the communication part 222 may transmit a command signal of the control part 224 to the oral scanner 110 and may receive image information of a target oral structure (that is, oral scan data, an oral scan position, an oral scan orientation, etc.) from the oral scanner 110.

The control part 224 may control the oral scanner 110 to capture an image of the target oral structure. Specifically, the control part 224 may control a light source part installed in the oral scanner 110 to radiate light toward at least one of a plurality of optical systems. Also, the control part 224 may control an image sensor part installed in the oral scanner 110 to detect light reflected by at least one of the plurality of optical systems. The control part 224 may control the image sensor to obtain two stereo images from an image of the detected light. The control part 224 may control the display part 226 to display the two stereo images obtained from the image sensor part. Also, the control part 224 may control three-dimensional oral structure model data calculated from the two stereo images to be visualized and displayed on the display part 226.

In one embodiment, the control part 224 may generate a visual guide. Specifically, the control part 224 may obtain first oral scan data from the oral scanner 110 through the communication part 222. Also, the control part 224 may calculate scan completeness of a plurality of regions of the first oral scan data and may determine a supplementation-required region among the plurality of regions. Here, a region of which scan completeness is lower than a predetermined threshold value may be determined as the supplementation-required region. Additionally, the supplementation-required region is a region that needs an additional scan, and the control part 224 may calculate the position and orientation of the oral scanner 110 that are required for performing an additional scan for the supplementation-required region. The control part 224 may generate a visual guide based on first scan completeness of the supplementation-required region and the calculated position and orientation of the oral scanner 110. Here, the first scan completeness of the supplementation-required region may indicate completeness of an oral model for the supplementation-required region that is obtained by the oral scanner 110. The scan completeness of supplementation-required region can be improved through an additional scan.

In one embodiment, the visual guide may include a geometrical shape that indicates at least one of the first scan completeness of the supplementation-required region, the calculated position of the oral scanner 110, and the calculated orientation of the oral scanner 110. Also, depending on an embodiment, the visual guide may include a line that indicates at least one of the calculated position of the oral scanner 110 and the calculated orientation of the oral scanner 110. The control part 224 may send the generated visual guide and the supplementation-required region to the display part 226.

In one embodiment, the control part 224 may control a surface size of the geometrical shape of the visual guide. Specifically, the control part 224 may obtain second oral scan data for the supplementation-required region from the oral scanner 110 through the communication part 222. Also, the control part 224 may calculate second scan completeness of the supplementation-required region based on the second oral scan data. Additionally, the control part 224 may change, in real time, the surface size of the geometrical shape of the visual guide that is associated with the supplementation-required region. That is, the control part 224 may change the surface size of the geometrical shape based on the size of the supplementation-required region in real time.

In one embodiment, the control part 224 may control a color of the geometrical shape of the visual guide. Specifically, the control part 224 may obtain second oral scan data for the supplementation-required region from the oral scanner 110 through the communication part 222. Also, the control part 224 may calculate third scan completeness of the supplementation-required region based on the third oral scan data. Additionally, the control part 224 may change, in real time, the color of the geometrical shape of the visual guide that is associated with the supplementation-required region.

In one embodiment, the control part 224 may visualize the supplementation-required region. In this case, the control part 224 may determine at least one of the type, color, and pattern of the geometrical shape depending on the type of the supplementation-required region. Here, the type of the supplementation-required region may include a void region, a contradictory region, a change region, etc. Accordingly, the control part 224 may send a visual guide corresponding to each type of the supplementation-required region to the display part 226.

In one embodiment, the control part 224 may visualize a plurality of teeth based on oral scan data. Also, the control part 224 may generate an outline of each of the plurality of visualized teeth. Here, a color of the outline of each of the plurality of teeth may be determined based on scan completeness associated with a region of each of the plurality of teeth. The control part 224 may send the plurality of visualized teeth and the generated outline of each of the plurality of teeth to the display part 226.

In one embodiment, the control part 224 may adjust transparency of the supplementation-required region. Specifically, the control part 224 may receive a first user input selecting the type of the supplementation-required region. Also, the control part 224 may receive a second user input selecting transparency of the supplementation-required region. Based on the first user input and the second user input, the control part 224 may change transparency of the supplementation-required region of which the type corresponds to the first user input to a value corresponding to the second user input. Additionally, the control part 224 may change transparency of the remaining teeth not including the supplementation-required region of which the type corresponds to the first user input to a predefined value (for example, 100% transparency). The control part 224 may control the display part 226 to change transparency of the corresponding supplementation-required region.

In one embodiment, the control part 224 may receive the current position and orientation of the oral scanner 110 through the communication part 222. Also, the control part 224 may generate a geometrical shape that corresponds to the current position and orientation of the oral scanner 110. The control part 224 may control the display part 226 so that the generated geometrical shape is output together with the visual guide.

The display part 226 may display information and/or data received from the oral scanner 110 or the control part 224 using images. In this case, the images displayed on the display part 226 may include two stereo images or three-dimensional oral structure model images. In one embodiment, the display part 226 may include a display panel device such as a light-emitting diode (LED) display, an organic light-emitting diode (OLED) display, a liquid crystal display (LCD), or a touch display.

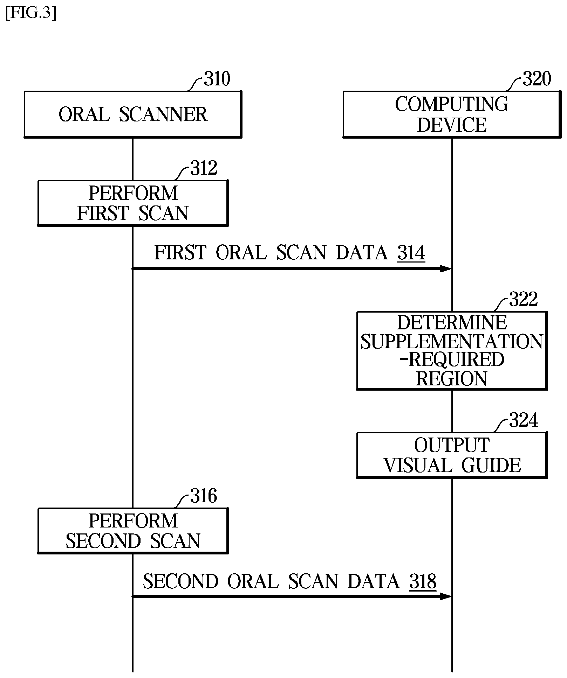

FIG. 3 is a flowchart showing an example of a method of providing a visual guide according to one embodiment of the present disclosure. In one embodiment, an oral scanner 310 may perform a first oral scan (312). Also, the oral scanner 310 may send first oral scan data 314 to a computing device 320.

In one embodiment, the computing device 320 may calculate scan completeness of a plurality of regions of the first oral scan data 314. Also, based on the calculated scan completeness, the computing device 320 may determine a supplementation-required region among the plurality of regions (322). Additionally, the computing device 320 may calculate the position and orientation of the oral scanner relative to the supplementation-required region.

In one embodiment, based on first scan completeness of the supplementation-required region, the calculated position of the oral scanner, and the calculated orientation of the oral scanner, the computing device 320 may generate a visual guide and output the visual guide on a display (324). Here, the visual guide may include a geometrical shape that indicates at least one of the first scan completeness of the supplementation-required region, the calculated position of the oral scanner, and the calculated orientation of the oral scanner. For example, when the geometrical shape is a circular shape, the position of the circular shape output on the display may indicate the calculated position of the oral scanner, and the orientation orthogonal to the circular shape may indicate the calculated orientation of the oral scanner.

Although it has been described above that the visual guide is formed as a geometrical shape, when the supplementation-required region is projected on a specific reference plane, the shape of the visual guide may be determined to be the shape formed on the specific reference plane. Therefore, the shape of the visual guide may be similar to the two-dimensional shape of the supplementation-required region. At this time, the position and normal orientation of the reference plane may be determined according to a distance from a target tooth requiring a supplementary scan and a scan orientation. The following description of an embodiment in which the visual guide is formed as a geometrical shape may, of course, be applied to the visual guide formed in the shape similar to the two-dimensional shape of the supplementation-required region.

In one embodiment, the size of the geometrical shape may be determined based on the size of the supplementation-required region. For example, the supplementation-required region is a region that requires an additional scan due to low scan completeness, and the larger the size of the supplementation-required region, the larger the size of the geometrical shape may be. Accordingly, when scan completeness of the supplementation-required region is improved through an additional scan, as the size of the corresponding supplementation-required region decreases, the size of the geometrical shape may also decrease.

In one embodiment, a color of the geometrical shape may be determined based on scan completeness of the supplementation-required region. For example, when the density of additionally required data is high due to low scan completeness of the supplementation-required region, the geometrical shape may be shown in red. In another example, when the density of additionally required data is low due to high scan completeness of the supplementation-required region, the geometrical shape may be shown in yellow. Alternatively, the color of the geometrical shape may be determined based on the type of the supplementation-required region. For example, when the supplementation-required region is a void region, the geometrical shape may be shown in black that corresponds to the void region.

In one embodiment, the oral scanner 310 may perform a second oral scan (316). Also, the oral scanner 310 may send second oral scan data 318 to the computing device 320. Additionally, the computing device 320 may calculate second scan completeness of the supplementation-required region based on the second oral scan data 318. In this case, the size of the geometrical shape output on the display may change in real time based on the second scan completeness. Accordingly, using the visual guide, the user can check, in real time, whether an additional scan is being appropriately performed. Also, the color of the geometrical shape output on the display may change in real time based on the second scan completeness. Accordingly, when scan completeness of the supplementation-required region is improved through an additional scan, the color of the geometrical shape may change.

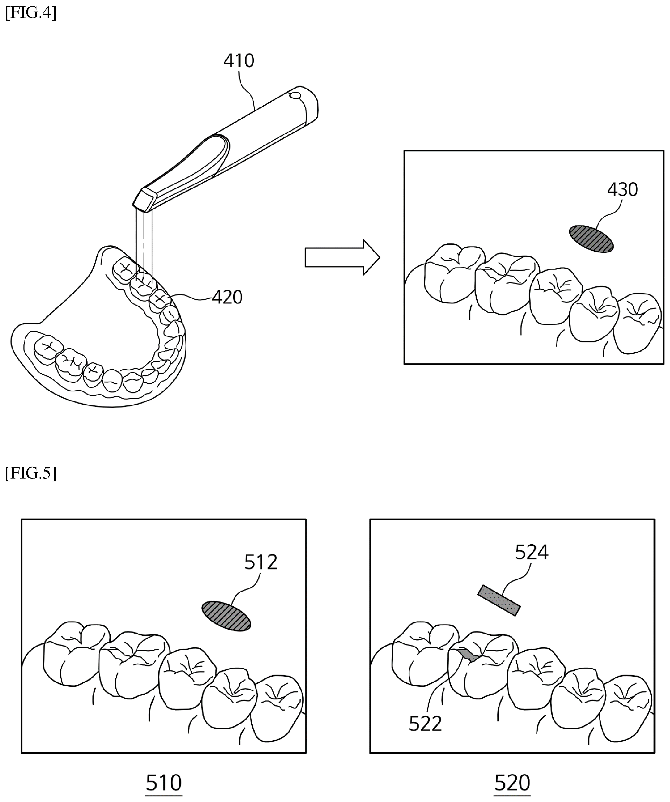

FIG. 4 shows an example of outputting a visual guide 440 according to one embodiment of the present disclosure. In one embodiment, an oral scanner 410 may generate first oral scan data by being inserted into an oral cavity of a dental patient by a dentist and scanning teeth 420 in a non-contact manner. Also, the oral scanner 410 may send the first oral scan data to an external computing device (not illustrated).

In one embodiment, the computing device may calculate scan completeness of a plurality of regions based on the first oral scan data. Here, the scan completeness of the plurality of regions may mean scan completeness of each of the plurality of regions in an oral cavity. When the scan completeness is low, distortion of the oral scan data may occur, and thus inaccurate treatment and care may be performed.

In one embodiment, based on the calculated scan completeness, a supplementation-required region that requires an additional scan may be determined among the plurality of regions. Here, types of the supplementation-required region may include a void region (for example, a region from which oral scan data is lost), a contradictory region (for example, a region where overlapping surfaces of multiple intraoral images fail to match), a change region (for example, a region where there is a change in a patient's teeth compared to previous oral scan data of the same patient), a region where foreign matter is present, a region where an unclear boundary line is present, etc.

In one embodiment, the computing device may calculate the position and orientation of the oral scanner relative to the supplementation-required region. Here, the position and orientation of the oral scanner relative to the supplementation-required region may mean the position and orientation of the oral scanner for performing an additional oral scan. The position and orientation of the oral scanner may be determined through various algorithms that calculate the position and orientation at and in which additional scanning is required for a region of which scan completeness is lower than a predetermined value.

In one embodiment, the computing device may output, on a display, the visual guide 430 generated based on first scan completeness of the supplementation-required region and the calculated position and orientation of the oral scanner. Here, the visual guide 430 may include a geometrical shape that indicates at least one of the first scan completeness of the supplementation-required region, the calculated position of the oral scanner, and the calculated orientation of the oral scanner. Also, the geometrical shape may show the shape of a two-dimensional figure or a three-dimensional figure. Additionally, the computing device may visualize the supplementation-required region in a pattern, a color, etc., and may output the supplementation-required region on the display together with the visual guide 430.

In one embodiment, the visual guide 430 may indicate scan completeness of additional oral scan data. Specifically, by the teeth 420 being additionally scanned by the dentist, the computing device may obtain second oral scan data for the supplementation-required region from the oral scanner 410. Also, second scan completeness of the supplementation-required region may be calculated based on the second oral scan data. In this case, the size of a surface included in the visual guide 430 may change in real time based on the second scan completeness. For example, when the scan completeness of the supplementation-required region increases due to repeatedly performing an additional oral scan, the size of the surface may gradually decrease. That is, the size of the surface may correspond to the size of the supplementation-required region. Accordingly, when the scan completeness of the corresponding supplementation-required region has become sufficiently high, the visual guide 430 may be removed. Alternatively, the color of the surface included in the visual guide 430 may change in real time based on the second scan completeness. For example, when the scan completeness of the supplementation-required region increases due to performing an additional oral scan, the color of the surface may change.

With such a configuration, the user can conveniently check an intraoral region that requires an additional scan due to low scan completeness. Also, the user can easily check the type of supplementation-required region through a visual guide. Accordingly, the user can obtain more precise oral scan data by performing an additional scan.

FIG. 5 is a view showing examples of visual guides 512 and 524 according to one embodiment of the present disclosure. In one embodiment, a visual guide may include a geometrical shape that indicates at least one of first scan completeness of a supplementation-required region, a calculated position of an oral scanner, and a calculated orientation of the oral scanner. Additionally, at least one of the type, color, and pattern of the geometrical shape of the visual guide may be determined based on the type of the supplementation-required region.

A first example 510 shows an example in which the visual guide 512 is output on a display. The visual guide 512 may indicate the position and orientation of the oral scanner relative to the supplementation-required region. For example, when the visual guide 512 has a circular shape, the position of the circular shape may indicate the position of the oral scanner relative to the supplementation-required region. Also, the orientation orthogonal to the plane of the circular shape may indicate the orientation of the oral scanner relative to the supplementation-required region.

In one embodiment, the visual guide 512 may include a geometrical shape that corresponds to the type of the supplementation-required region. For example, a geometrical shape with a circular surface may indicate that the supplementation-required region is a void region. In another example, a geometrical shape whose surface has a stripe pattern may indicate that the supplementation-required region is a void region. In this case, the same stripe pattern may also be displayed on the supplementation-required region. Also, an area of the geometrical shape may be determined based on the scan completeness of the supplementation-required region or the size of the supplementation-required region.

A second example 520 shows an example in which a supplementation-required region 522 and the visual guide 524 are output on a display. The visual guide 524 may indicate the position and orientation of the oral scanner relative to the supplementation-required region 522. Also, the visual guide 524 may include a geometrical shape that corresponds to the type of the supplementation-required region 522. For example, a geometrical shape with a quadrangular surface may indicate that the supplementation-required region 522 is a contradictory region. In another example, a geometrical shape whose surface has a dot pattern may indicate that the supplementation-required region 522 is a contradictory region.

In one embodiment, the supplementation-required region 522 may be visualized and output on a display. In this case, the supplementation-required region 522 may include the same pattern as the visual guide 524. Also, the supplementation-required region 522 may be determined based on the scan completeness of the corresponding region. For example, when the scan completeness is lower than a predetermined threshold value, the corresponding region may be determined as the supplementation-required region.

Although it is illustrated in FIG. 5 that the shape and pattern of the surface of the geometrical shape are determined based on the type of the supplementation-required region, the present disclosure is not limited thereto. For example, the type of the supplementation-required region may also be determined through the color of the geometrical shape. In this case, the supplementation-required region may also be shown in the same color.

With such a configuration, just by looking at the geometrical shape, the user can easily recognize for which reason the corresponding region requires supplementation. Also, from the surface size of the geometrical shape, the user can predict until when additional scanning should be performed. Accordingly, the user can easily perform an additional oral scan for a patient and can obtain high-quality oral scan data.

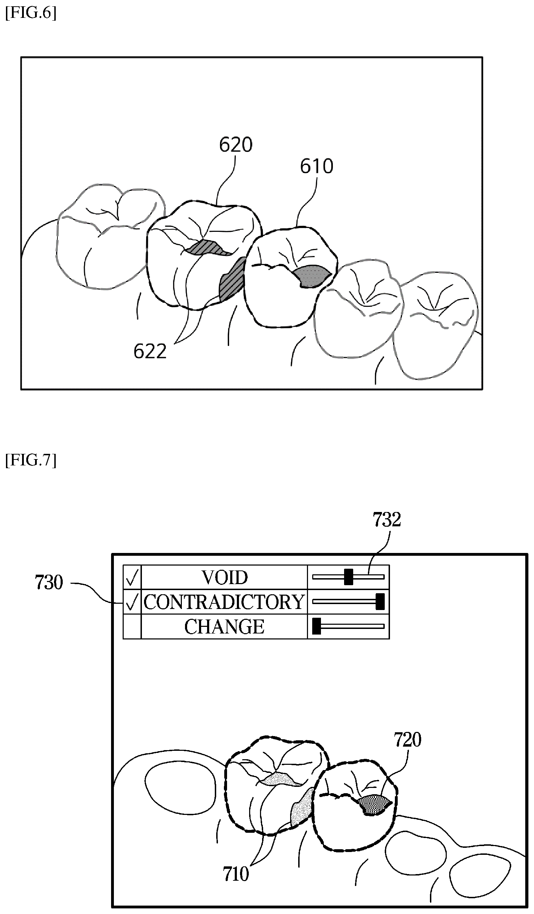

FIG. 6 is a view showing an example of outputting outlines of teeth according to one embodiment of the present disclosure. In one embodiment, a plurality of teeth in an oral cavity and an outline of each of the plurality of teeth may be generated. In this case, the generated plurality of teeth and outline of each of the plurality of teeth may be visualized and output on a display.

In one embodiment, the color of the outline may be determined based on scan completeness associated with a region of each of the plurality of teeth. For example, when a supplementation-required region 622 is present on a first tooth, a first outline 620 of the first tooth may be shown in blue. On the other hand, teeth where a supplementation-required region is not present due to sufficiently high scan completeness may be shown in red.

In one embodiment, the color of the outline may be determined based on the type of the supplementation-required region. For example, when the supplementation-required region 622 of the first tooth is a void region, the first outline 620 may be shown in blue. On the other hand, when a supplementation-required region of a second tooth is a contradictory region, a second outline 610 may be shown in yellow.

In one embodiment, the type of the outline may be determined based on the scan completeness associated with the region of each of the plurality of teeth. For example, an outline of a tooth where a supplementation-required region is present may be shown as a dotted line. On the other hand, an outline of a tooth where a supplementation-required region is not present due to sufficiently high scan completeness may be shown as a solid line.

Although only the outlines of the teeth are illustrated in FIG. 6, the present disclosure is not limited thereto, and the above-described visual guides may be output together on the display.

With such a configuration, the user can easily identify a tooth that requires an additional scan. Also, from a color or the like of an outline, which type of supplementation-required region is present on a corresponding tooth can be easily checked.

FIG. 7 is a view showing an example of adjusting transparency of some teeth according to one embodiment of the present disclosure. In one embodiment, the user may adjust transparency of a supplementation-required region through a transparency adjustment interface 730. Specifically, the user may select the type of supplementation-required region in the transparency adjustment interface 730. Also, the user may set the transparency of the supplementation-required region using a sliding bar 732.

For example, the user may check a void region and a contradictory region in the transparency adjustment interface 730, and then may adjust the sliding bar 732 corresponding to each supplementation-required region to adjust transparency of supplementation-required regions 710 and 720. For example, the user may set a change region to be invisible, set the contradictory region 720 to be clearly visible, and set the void region 710 to be moderately transparent.

In one embodiment, the user may set teeth not including a supplementation-required region to be transparent. Specifically, when the user has selected the type of supplementation-required region in the transparency adjustment interface 730, teeth not including the selected supplementation-required region may be set to be transparent.

Although FIG. 7 illustrates that transparency of a supplementation-required region is set using a sliding bar, the present disclosure is not limited thereto. For example, the user may also set transparency of a supplementation-required region by inputting a numerical value of the transparency of the supplementation-required region.

With such a configuration, by processing teeth that are not subject to scanning to be transparent, the user can focus more on a tooth that requires an additional scan. Also, by setting transparency for each type of supplementation-required region, the user can perform an additional scan while unnecessary supplementation-required regions are filtered out.

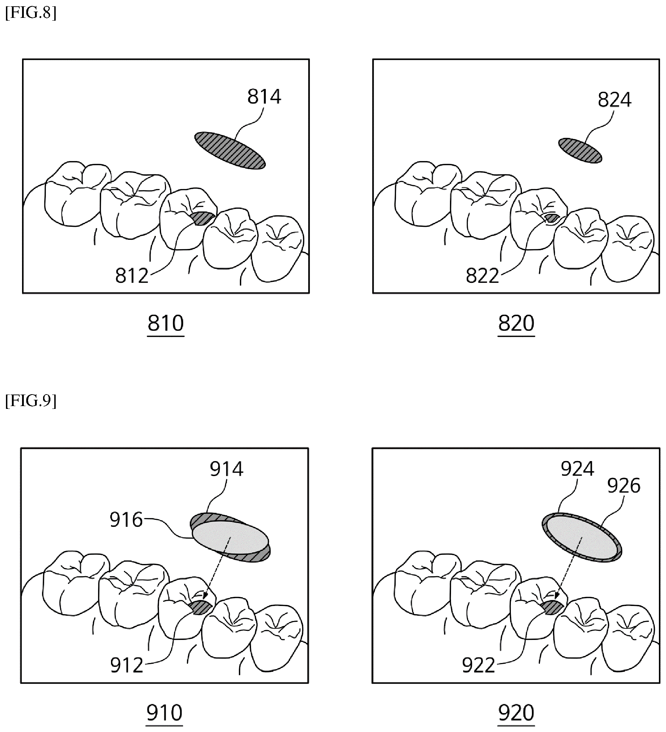

FIG. 8 is a view showing an example of changing the size of a geometrical shape according to one embodiment of the present disclosure. A first screen 810 shows an example in which a first visual guide 814 for a first supplementation-required region 812 is output on a display. In one embodiment, a computing device may obtain first oral scan data from an oral scanner. As described above with reference to FIGS. 3 to 5, based on the first oral scan data, the first supplementation-required region 812 and the first visual guide 814 for an additional scan may be visualized and output on the display. Here, the first visual guide 814 may include a geometrical shape, and the size of the geometrical shape may be determined based on the size of the first supplementation-required region 812.

A second screen 810 shows an example in which a second visual guide 824 for a second supplementation-required region 822 is output on a display. In one embodiment, a computing device may obtain second oral scan data (that is, data obtained through an additional scan) from an oral scanner. In this case, by supplementing data through an additional scan, the second supplementation-required region 822 may be determined again among a plurality of regions. Also, as the size of the second supplementation-required region 822 decreases, the size of a geometrical shape of the second visual guide 824 may decrease. Such a change in the size of the geometrical shape may be performed in real time while an additional scan is being performed by the user. Accordingly, when scan completeness becomes sufficiently high by the user performing an additional scan for the supplementation-required region, the visual guide indicating the corresponding region may be removed from the display.

Although FIG. 8 illustrates that, as the size of the second supplementation-required region 822 decreases, the size of the geometrical shape of the second visual guide 824 decreases while the geometrical shape maintains the same shape (that is, a circular shape), the present disclosure is not limited thereto. For example, when a portion of the supplementation-required region is reduced through an additional scan, a portion of the geometrical shape of the visual guide that corresponds to the reduced portion of the supplementation-required region may also be reduced. Accordingly, the shape of the reduced geometrical shape may be different from the shape of the previous geometrical shape.

FIG. 9 is a view showing an example of outputting together a visual guide indicating the current position and orientation of the oral scanner and a visual guide indicating a target position and orientation of the oral scanner on a display according to one embodiment of the present disclosure. A first screen 910 shows an example in which a first visual guide 914 for a supplementation-required region 912 and a second visual guide 916 indicating the current position and orientation of the oral scanner are output on a display. In one embodiment, the first visual guide 914 may indicate a target position and orientation of the oral scanner for performing an additional scanning task for the supplementation-required region 912. That is, the first visual guide 914 may indicate the position and orientation where the oral scanner will start additional scanning in order to improve scan completeness of the supplementation-required region 912. Also, a computing device may receive data associated with the current position and orientation of the oral scanner from the oral scanner. In this case, based on the current position and orientation of the oral scanner, the computing device may generate the second visual guide 916 indicating the current position and orientation of the oral scanner.

In one embodiment, the second visual guide 916 may correspond to the first visual guide 914. Specifically, the second visual guide 916 may have the same shape as the first visual guide 914. Also, the position of the second visual guide 916 may indicate the current position of the oral scanner, and the orientation orthogonal to the second visual guide 916 may indicate the current orientation of the oral scanner.

A second screen 920 shows an example in which the center of a first visual guide 924 for a supplementation-required region 922 and the center of a second visual guide 926 indicating the current position and orientation of the oral scanner coincide. In one embodiment, the user may manipulate the oral scanner according to the first visual guide 924. In this case, when the second visual guide indicating the current position and orientation of the oral scanner matches the first visual guide 924, it may mean that an additional scan is being appropriately performed. In this way, when an additional scan is being appropriately performed, the center of the second visual guide 926 may coincide with the center of the first visual guide 924, and the second visual guide 926 and the first visual guide 924 may be coplanar. Accordingly, the user may check the current position and orientation of the oral scanner from comparison with the first visual guide 924.

Although FIG. 9 illustrates that the shape and size of the first visual guide and the shape and size of the second visual guide are different, the present disclosure is not limited thereto, and the shapes and sizes of the first visual guide and the second visual guide may be the same.

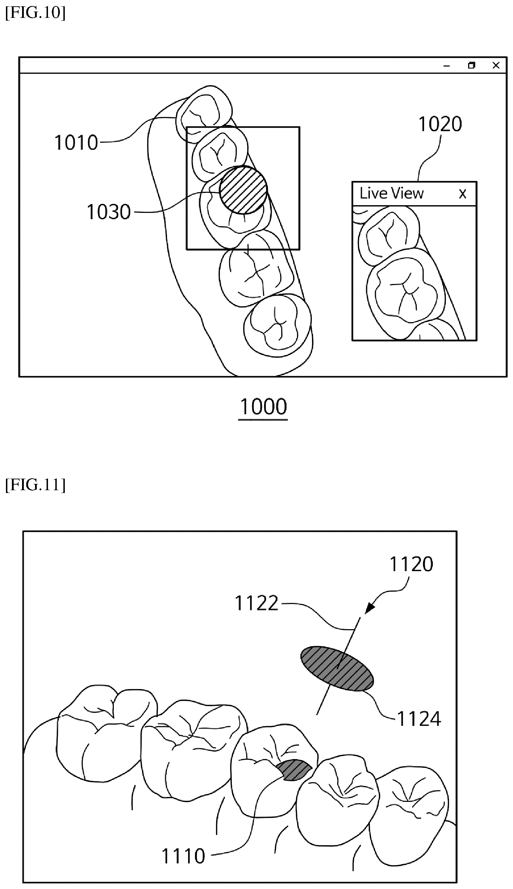

FIG. 10 is a view showing an example of a user interface screen 1000 on which a visual guide 1030 is output according to one embodiment of the present disclosure. In one embodiment, the user may check a three-dimensional oral model 1010, a live view 1020, and the visual guide 1030 through the user interface screen 1000. Here, the live view 1020 may include an image captured at and in the current position and orientation of an oral scanner. Also, the three-dimensional oral model 1010 may include data that is visualized based on oral scan data obtained from the oral scanner.

In one embodiment, the color of an outline of a geometrical shape of the visual guide 1030 may be determined based on the current position or orientation of the oral scanner. Specifically, when the current position of the oral scanner and the position of the geometrical shape match, the color of the outline of the geometrical shape may be a predetermined color (for example, red). Also, when the current orientation of the oral scanner and the orientation orthogonal to the geometrical shape are parallel, the color of the outline of the geometrical shape may be a predetermined color (for example, yellow). Additionally, when the current position and orientation of the oral scanner match the position of the geometrical shape and the orientation orthogonal to the geometrical shape, the color of the outline of the geometrical shape may be a predetermined color (for example, blue).

In one embodiment, from the shape of the geometrical shape of the visual guide 1030, the user may check whether the current orientation of the oral scanner is appropriate. For example, when the geometrical shape is a circular shape, the geometrical shape may be shown as an elliptical shape due to the three-dimensional oral model 1010 being rotated in the current orientation of the oral scanner. At this time, the user may adjust the current orientation of the oral scanner to the orientation orthogonal to the geometrical shape, thereby allowing the geometrical shape to be shown in a circular shape instead of an elliptical shape. Accordingly, by checking the geometrical shape that is a circular shape, the user may determine that the current orientation of the oral scanner is appropriate.

Although FIG. 10 illustrates that the user interface screen 1000 only includes the three-dimensional oral model 1010 and the live view 1020, the present disclosure is not limited thereto, and the user interface screen 1000 may further include a menu, an icon, a screen, etc., for supporting various functions.

With such a configuration, from a color of an outline of a visual guide, the user can easily determine whether the current position and orientation of an oral scanner are appropriate. Accordingly, an additional oral scan can be conveniently performed.

FIG. 11 is a view showing an example of outputting a visual guide 1120 according to one embodiment of the present disclosure. In one embodiment, the visual guide 1120 may include not only a geometrical shape indicating at least one of scan completeness of a supplementation-required region 1110, a calculated position of an oral scanner, and a calculated orientation of the oral scanner, but also a line 1122 indicating at least one of the calculated position of the oral scanner and the calculated orientation of the oral scanner. Here, one end of the line 1122 that is located in the opposite direction from teeth may indicate the calculated position of the oral scanner. Alternatively, an intermediate point of the line 1122 may indicate the calculated position of the oral scanner. Also, the orientation parallel to the line 1122 may indicate the calculated orientation of the oral scanner.

In one embodiment, the geometrical shape may include a surface 1124 orthogonal to the line 1122. Here, the surface 1124 may be located at the intermediate point of the line. Also, at least one of the type (for example, circular, quadrangular, etc.), color, and pattern of the surface 1124 may be associated with the type of the supplementation-required region. For example, the visual guide 1120 may show that the supplementation-required region is a void region through the circular shape of the surface 1124. In another example, the visual guide 1120 may show that the supplementation-required region is a void region through a pattern of the surface 1124.

Alternatively, the color of the surface 1124 may be associated with the scan completeness of the supplementation-required region. For example, when the density of additionally required data is high due to low scan completeness of the supplementation-required region 1110, the surface 1124 may be shown in red. In another example, when the density of additionally required data is low due to high scan completeness of the supplementation-required region 1110, the surface 1124 may be shown in yellow.

In one embodiment, the size of the surface 1124 may be determined based on the size of the supplementation-required region 1110. For example, the larger the size of the supplementation-required region 1110, the larger the size of the surface 1124 may be. Accordingly, when scan completeness of the supplementation-required region 1110 increases through an additional scan, and the size of the supplementation-required region 1110 decreases, the size of the surface 1124 may also decrease.

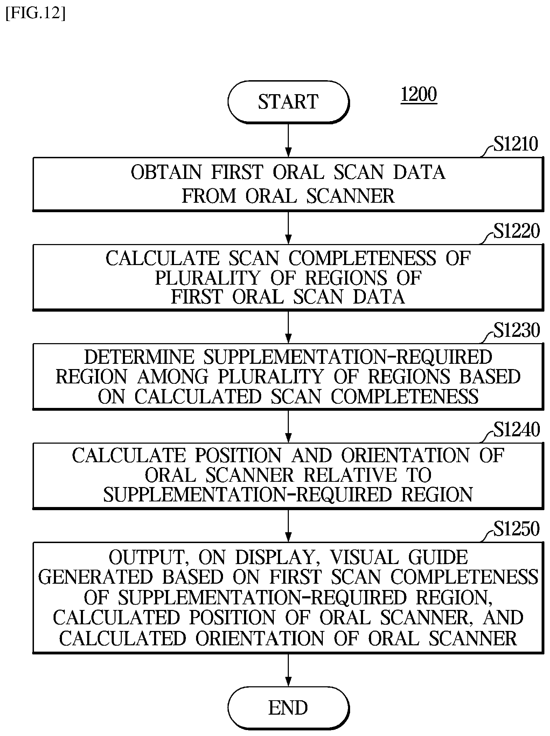

FIG. 12 is a flowchart showing an example of a method 1200 according to one embodiment of the present disclosure. In one embodiment, the method 1200 may be performed by at least one processor. The method 1200 may start by the processor obtaining first oral scan data from an oral scanner (S1210). Next, the processor may calculate scan completeness of a plurality of regions of the first oral scan data (S1220).

Next, the processor may determine a supplementation-required region among the plurality of regions based on the calculated scan completeness (S1230). Also, the processor may calculate the position and orientation of the oral scanner relative to the supplementation-required region (S1240).

Next, the processor may output, on a display, a visual guide generated based on first scan completeness of the supplementation-required region, the calculated position of the oral scanner, and the calculated orientation of the oral scanner (S1250). Here, the visual guide may include a geometrical shape that indicates at least one of the first scan completeness of the supplementation-required region, the calculated position of the oral scanner, and the calculated orientation of the oral scanner. In this case, the color of an outline of the geometrical shape may be determined based on the current position or orientation of the oral scanner. Also, the visual guide may include a first visual guide that indicates the current position or orientation of the oral scanner relative to the supplementation-required region and a second visual guide that indicates a target position or orientation of the oral scanner for performing additional scanning for the supplementation-required region.

In one embodiment, the processor may visualize the supplementation-required region and output the supplementation-required region on the display. Here, at least one of the type, color, and pattern of the geometrical shape may be determined based on the type of the supplementation-required region. Also, the type of the supplementation-required region may include at least one of a void region, a contradictory region, and a change region.

In one embodiment, the visual guide may further include a line that indicates at least one of the calculated position of the oral scanner and the calculated orientation of the oral scanner, and the geometrical shape may include a surface orthogonal to the line. In this case, the size of the surface may be determined based on the size of the supplementation-required region.

In one embodiment, the processor may obtain second oral scan data for the supplementation-required region from the oral scanner. Also, the processor may calculate second scan completeness of the supplementation-required region based on the second oral scan data. Here, the size of the geometrical shape may change in real time based on the second scan completeness.

In one embodiment, the processor may visualize a plurality of teeth in an oral cavity and an outline of each of the plurality of teeth that are generated based on the first oral scan data and may output the plurality of teeth and the outline of each of the plurality of teeth on the display. Here, the color of the visualized outline may be determined based on the scan completeness associated with a region of each of the plurality of teeth.

In one embodiment, the processor may receive a first user input selecting the type of the supplementation-required region. Also, the processor may receive a second user input selecting transparency of the supplementation-required region. In response to receiving the first user input and the second user input, the processor may change transparency of the supplementation-required region of which the type corresponds to the first user input to a value corresponding to the second user input and may output the value on the display. Additionally, the processor may change transparency of the remaining teeth not including the supplementation-required region of which the type corresponds to the first user input among the plurality of teeth to a predefined value and may output the value on the display.

In one embodiment, the geometrical shape may include a two-dimensional figure. In this case, the position of the two-dimensional figure may indicate the calculated position of the oral scanner. Also, the orientation orthogonal to the two-dimensional figure may indicate the calculated orientation of the oral scanner. Additionally, the size of the two-dimensional figure may be determined based on the size of the supplementation-required region. The color of the two-dimensional figure may be determined based on the scan completeness of the supplementation-required region.

In one embodiment, the processor may obtain third oral scan data for the supplementation-required region from the oral scanner. Also, the processor may calculate third scan completeness of the supplementation-required region based on the third oral scan data. Here, the color of the geometrical shape may change in real time based on the third scan completeness.

The above-described method may be provided as a computer program stored in a computer-readable recording medium to be executed by a computer. Media may continuously store executable programs on a computer or may temporarily store the executable programs for execution or download. Furthermore, the media may be a variety of recording or storage devices in the form of a combination of a single or several pieces of hardware, and is not limited to media directly connected to a certain computer information-processing system and may be distributed over a network. Media may include, for example, magnetic media such as hard disks, floppy disks, or magnetic tapes, optical recording media such as CD-ROMs or DVDs, magneto-optical media such as floptical disks, a ROM, a RAM, a flash memory, and so on, and may be configured to store program instructions. Also, other examples of media may include recording media or storage media managed by app stores that distribute applications or sites, servers, etc., that supply or distribute various other pieces of software.

The methods, operations, or techniques of the present disclosure may also be implemented by various means. For example, the techniques may also be implemented by hardware, firmware, software, or a combination thereof. Those of ordinary skill in the art will understand that the various illustrative logical blocks, modules, circuits, and algorithm steps described in connection with the present disclosure may be implemented by electronic hardware, computer software, or combinations of both. To clearly describe the interchange of hardware and software, various illustrative components, blocks, modules, circuits, and steps are described above generally in terms of function. Whether such function is implemented by hardware or software depends on specific application and design requirements provided on the overall information processing system. Those of ordinary skill in the art may implement the functions described in various ways for each particular application, but such implementations should not be interpreted to be outside the scope of the present disclosure.

In hardware implementations, the processing units used to perform techniques may also be implemented within one or more application-specific integrated circuits (ASICs), digital signal processors (DSPs), GPUs, digital signal processing devices (DSPDs), programmable logic devices (PLDs), field programmable gate arrays (FPGAs), processors, controllers, microcontrollers, microprocessors, electronic devices, other electronic units designed to perform the functions described in the present disclosure, computers, or combinations thereof.

When implemented in software, the techniques may also be stored in or transmitted through a computer-readable medium as one or more instructions or codes. Computer-readable media may include both computer storage media and communication media, in addition to any medium that facilitates transfer of a computer program from one place to another place. Storage media may also be any available media that may be accessed by a computer. By way of nonlimiting examples, such computer-readable media may include a RAM, a ROM, an EEPROM, a CD-ROM or another optical disc storage, a magnetic disk storage or other magnetic storage devices, or any other medium that may be used to transfer or store the desired program codes in the form of instructions or data structures and may be accessed by a computer. Also, any connection may suitably be made by a computer-readable medium.

Although the above-described embodiments are described as utilizing aspects of the presently disclosed subject matter in one or more standalone computer information processing systems, the present disclosure is not limited thereto and may also be implemented in conjunction with any computing environment, such as a network or distributed computing environment. Furthermore, aspects of the subject matter of the present disclosure may also be implemented in multiple processing chips or devices, and storages may also be similarly affected by multiple devices. Such devices may include personal computers (PCs), network servers, and portable devices.

Although the present disclosure has been described herein with respect to some embodiments thereof, various modifications and variations may be made thereto within the scope not departing from the scope of the present disclosure that can be understood by those of ordinary skill in the art to which the invention of the present disclosure pertains. Also, such modifications and variations should be considered as falling within the scope of the appended claims.
110: oral scanner
120: computing device

## Claims

1. A method of providing a visual guide for an oral scanner that is performed by at least one processor, the method comprising:
obtaining first oral scan data from an oral scanner;
calculating scan completeness of a plurality of regions of the first oral scan data;
determining a supplementation-required region among the plurality of regions based on the calculated scan completeness;
calculating a position and an orientation of the oral scanner relative to the supplementation-required region; and
outputting, on a display, a visual guide generated based on first scan completeness of the supplementation-required region, the calculated position of the oral scanner, and the calculated orientation of the oral scanner.

2. The method of claim 1, wherein the visual guide includes a geometrical shape that indicates at least one of the first scan completeness of the supplementation-required region, the calculated position of the oral scanner, and the calculated orientation of the oral scanner.

3. The method of claim 2, wherein the visual guide further includes a line that indicates at least one of the calculated position of the oral scanner and the calculated orientation of the oral scanner, and the geometrical shape includes a surface orthogonal to the line.

4. The method of claim 2, further comprising:
obtaining second oral scan data for the supplementation-required region from the oral scanner; and
calculating second scan completeness of the supplementation-required region based on the second oral scan data,
wherein a size of the geometrical shape changes in real time based on the second scan completeness.

5. The method of claim 2, further comprising visualizing the supplementation-required region and outputting the visualized supplementation-required region on the display,
wherein at least one of a type, a color, and a pattern of the geometrical shape is determined based on a type of the supplementation-required region, and
the type of the supplementation-required region includes at least one of a void region, a contradictory region, and a change region.

6. The method of claim 1, further comprising visualizing a plurality of teeth in an oral cavity and an outline of each of the plurality of teeth that are generated based on the first oral scan data and outputting the plurality of teeth and the outline of each of the plurality of teeth on the display,
wherein a color of the visualized outline is determined based on scan completeness associated with a region of each of the plurality of teeth.

7. The method of claim 6, wherein visualizing the plurality of teeth and the outline of each of the plurality of teeth and outputting the plurality of teeth and the outline of each of the plurality of teeth on the display includes:
receiving a first user input selecting the type of the supplementation-required region;
receiving a second user input selecting transparency of the supplementation-required region; and
in response to receiving the first user input and the second user input, changing transparency of the supplementation-required region of which the type corresponds to the first user input to a value corresponding to the second user input and outputting the value on the display.

8. The method of claim 7, wherein visualizing the plurality of teeth and the outline of each of the plurality of teeth and outputting the plurality of teeth and the outline of each of the plurality of teeth on the display further includes:
changing transparency of remaining teeth not including the supplementation-required region of which the type corresponds to the first user input among the plurality of teeth to a predefined value and outputting the value on the display.

9. The method of claim 2, wherein:
the geometrical shape includes a two-dimensional figure;
a position of the two-dimensional figure indicates the calculated position of the oral scanner; and
an orientation orthogonal to the two-dimensional figure indicates the calculated orientation of the oral scanner.

10. The method of claim 9, wherein:
a size of the two-dimensional figure is determined based on a size of the supplementation-required region; and
a color of the two-dimensional figure is determined based on scan completeness of the supplementation-required region.

11. The method of claim 1, wherein the visual guide includes:
a first visual guide that indicates the current position or orientation of the oral scanner relative to the supplementation-required region; and
a second visual guide that indicates a target position or orientation of the oral scanner for performing additional scanning for the supplementation-required region.

12. The method of claim 2, further comprising:
obtaining third oral scan data for the supplementation-required region from the oral scanner; and
calculating third scan completeness of the supplementation-required region based on the third oral scan data,
wherein a color of the geometrical shape changes in real time based on the third scan completeness.

13. The method of claim 2, wherein a color of an outline of the geometrical shape is determined based on the current position or orientation of the oral scanner.

14. A device comprising:
a communication module;
a display;
a memory; and
at least one processor connected to the memory and configured to execute at least one program that is included in the memory and computer-readable,
wherein the at least one program includes instructions for:
obtaining first oral scan data from an oral scanner;
calculating scan completeness of a plurality of regions of the first oral scan data;
determining a supplementation-required region among the plurality of regions based on the calculated scan completeness;
calculating a position and an orientation of the oral scanner relative to the supplementation-required region; and
outputting, on the display, a visual guide generated based on first scan completeness of the supplementation-required region, the calculated position of the oral scanner, and the calculated orientation of the oral scanner.
